# EUROPEAN PATENT APPLICATION

(11) **EP 4 600 966 A1**
(43) Date of publication of application: **13.08.2025**
(21) Application number: 25156204.7
(22) Date of filing: 06.02.2025
(51) Int. Cl.: G16H 20/30, G16H 40/63, A61N 1/39, A61H 31/00

(54) **SYSTEMS, METHODS, AND APPARATUS FOR ADJUSTING MEDICAL TREATMENTS**

(30) Priority: 06.02.2024 US 202463550194 P; 30.01.2025 US 202519041899
(71) Applicant: Physio-Control, Inc., Redmond, WA 98052 (US)
(72) Inventor: PIRAINO, Daniel, Redmond, 98052 (US); STICKNEY, Ronald, Redmond, 98052 (US); STEWART, David, Redmond, 98052 (US); BEUNING, Gail, Redmond, 98052 (US)
(74) Representative: V.O.

(57) **Abstract**

A portable medical device for adjusting a cardiopulmonary resuscitation (CPR) treatment includes a processor. The processor is configured to determine a particular CPR treatment protocol from one or more CPR treatment protocols. The particular CPR treatment protocol may include a ratio of a number of chest compressions to ventilations for administering during a CPR cycle. The processor is configured to receive a CPR time period for the CPR treatment for the CPR treatment, to determine a number of CPR cycles for the CPR treatment based on the particular CPR treatment protocol, and to calculate a time duration of the CPR treatment based on the number of CPR cycles of the particular CPR treatment protocol. The processor is configured to compare the time duration to the CPR time period and to modify the particular CPR treatment protocol or CPR time period based on the comparison.

## Description

### CROSS-REFERENCE TO RELATED APPLICATION

The present application claims the benefit of U.S. Provisional Patent Application No. 63/550,194, filed February 6, 2024. The contents of which are hereby incorporated by reference in their entirety.

### FIELD

The present disclosure relates generally to medical devices and, more particularly, to systems and methods for adjusting medical treatments, such as cardiopulmonary resuscitation (CPR) treatments.

### BACKGROUND

This background description is provided for the purpose of generally presenting the context of the disclosure. Unless otherwise indicated herein, material described in this section is neither expressly nor impliedly admitted to be prior art to the present disclosure or the appended claims.

Cardiac arrest or arrhythmia can be a life-threatening medical condition in which a person's heart fails to provide adequate blood flow to support life. During a cardiac arrest, the electrical activity of the heart may be disorganized (ventricular fibrillation (VF)), too rapid (ventricular tachycardia (VT)), absent (asystole), or organized at a normal or slow heart rate. A person in cardiac arrest may first lose his or her pulse, then consciousness, and finally the ability to breath. These events can happen in a short period of time.

An effective treatment for cardiac arrest is to deliver or apply an electrical pulse using a device called an external defibrillator to defibrillate the heart. The electrical pulse generated by the external defibrillator may be applied across the patient's heart by means of electrodes or paddles placed on the body of the patient, such as the patient's chest, resulting in a flow of electrical current through the heart. The flow of electrical current is provided to halt the fibrillation, giving the heart a chance to start beating with a normal rhythm. The delivery of the electrical pulse, which is intended to return the heart to normal rhythm, is called defibrillation.

The probability of surviving cardiac arrest depends on the speed with which appropriate medical care is provided to a person experiencing cardiac arrest. Survival rates for cardiac arrest are the highest when defibrillation is conducted within the first few minutes after the onset of cardiac arrest. To decrease the time until appropriate medical care is provided, external defibrillators may be used to improve the overall success rate of treating persons in cardiac arrest.

An external defibrillator is typically a small, portable device that analyzes the heart's rhythm and prompts a user to deliver medical treatment to the person being treated. Once the external defibrillator is activated, it can guide a user or rescuer (e.g., a first responder) through each step of the treatment by providing audible and/or visual prompts that may include a cardiopulmonary resuscitation (CPR) treatment and/or a defibrillation treatment. The instructions provided by the external defibrillator to the user can improve the overall success rate of treating the person experiencing cardiac arrest.

There are generally two types of external defibrillators, manual external defibrillators and automated external defibrillators, or "AEDs." Manual external defibrillators are typically designed for use by medical professionals (e.g., emergency medical service workers) trained in using defibrillators. An AED is an external defibrillator with automated functions and user-friendly guidance that allows first responders (e.g., a firefighter or a police officer) or laypersons with minimal or no training to operate the defibrillator. In many cases, manual external defibrillators have an automatic, or AED, mode as well.

Using one of these types of external defibrillators, a user of the external defibrillator (e.g., a medical profession, a rescuer, or layperson) can deliver resuscitative treatments to a person experiencing cardiac arrest. For example, the external defibrillator may provide, based on CPR treatment protocols, instructions for performing cardiopulmonary resuscitation (CPR) treatments. The CPR treatments may include a sequence of chest compressions in order to improve blood flow to vital organs.

CPR treatment protocols for CPR treatments have been developed for external defibrillators that typically include instructions for performing CPR based on medical guidelines and standards. The external defibrillators may implement the CPR treatment protocols to provide instructions to user or rescuers (e.g., first responders) for performing CPR treatment. The CPR treatment protocols typically include a series of chest compressions, usually followed by one or more ventilations (e.g., a pattern or ratio of chest compressions to one or more ventilations). The CPR treatment protocols may vary depending on factors such as age classification of the person (e.g., adult or child/infant), an airway status (e.g., whether the person has his or her airway secured by artificial airway), whether the CPR is being delivered by one or two persons, and/or whether the person delivering CPR is a medical professional or a layperson.

Since different CPR treatment protocols may be used for different situations, the external defibrillator may allow a user or rescuer to input or select various parameters or factors to enable the external defibrillator to determine a CPR treatment protocol for the situation. Some external deliberators may also allow a user or rescuer to specify a CPR time period (e.g., a time period or interval to perform a CPR treatment). For example, a user or rescuer may input a CPR time period for a CPR treatment based on his or her training and/or techniques. Different first responder/EMS organizations may also have different protocols that they require first responders in their organization adhere to. However, when a user specifies a CPR time period, the time to implement the CPR treatment protocol by the external defibrillators may not conform to the user-specified CPR period. For example, the CPR treatment protocol selected by the external defibrillators may not be able to be completed or performed within the user-specified CPR period. As result, a portion of the CPR treatment may be cut-off or discarded. Further, a user-specified CPR period may be significantly longer than the time for the CPR treatment protocol to be implemented by the external defibrillators causing time gaps between successive CPR treatments. Thus, external defibrillators may not provide efficient and effective treatment for user-specified CPR periods, which may have a negative effect on patient outcome. Accordingly, it is desirable to develop systems and methods for adjusting CPR treatment protocols or CPR periods for CPR treatments.

### SUMMARY

The present application is directed to embodiments that relate to systems, methods, and apparatus for providing treatments to a person or patient experiencing a medical condition, such as cardiac arrest or any other cardiac rhythm abnormality. During an emergency episode, the embodiments may provide instructions to a user or rescuer for performing cardiac or resuscitation treatments, such as CPR treatments, on a person or patient. The embodiments may also be configured to deliver a defibrillation pulse or shock to the person to restore a normal heartbeat.

The embodiments may allow a user or rescuer to input or select information for a cardiac or cardiopulmonary resuscitation (CPR) treatment. The embodiments may also allow a user or rescuer to request or specify a CPR time period for a CPR treatment. Based on the user inputs, the embodiments may be configured to identify and select a CPR treatment protocol from one or more CPR treatment protocols. The CPR treatment protocol selected by the embodiments may include a pattern or ratio of a number of chest compressions to one or more ventilations for a CPR cycle (e.g., a number of chest compressions followed by one or more ventilations during a CPR cycle). The CPR treatment protocol may also include the rate at which the chest compressions should be delivered (e.g., compressions per minute) and the time over which the ventilations should be delivered or provided (e.g., each ventilation should have duration of about one second). The CPR treatment protocol may be consistent with guidelines provided by the American Heart Association or medical directors.

The embodiments may evaluate the selected CPR treatment protocol for the CPR treatment. Based on the evaluation, the embodiments may modify the selected CPR treatment protocol and/or modify the CPR time period inputted or selected by the user for the CPR treatment. For example, the embodiments may change the number of chest compressions for a CPR cycle, change the number of the ventilations for a CPR cycle, adjust a duration or time period of a ventilation, adjust the length or duration of a time period between ventilations, or adjust or modify the CPR time period inputted or selected by the user for the CPR treatment. The embodiments may adjust the CPR treatment protocol while remaining consistent and compliant with current medical guidelines and standards. Based on the modified CPR treatment protocol and/or the modified CPR time period inputted or selected by the user, the embodiments may provide rhythmic signals to guide a user in pacing and timing of chest compressions and may also provide signals to guide in the pacing and timing of ventilations for the CPR treatment.

In one aspect, a portable medical device for adjusting a cardiopulmonary resuscitation (CPR) treatment is disclosed. The portable medical device may comprise a user interface, a memory device, and a processor. The processor may be configured to determine a CPR treatment protocol from one or more CPR treatment protocols. The CPR treatment protocol may include a ratio of a number of chest compressions to one or more ventilations for administering during a CPR cycle. The processor may also be configured to receive a CPR time period for the CPR treatment, determine a number of CPR cycles for the CPR treatment based on the CPR treatment protocol, and calculate a time duration of the CPR treatment based on the number of CPR cycles of the CPR treatment protocol. Further, the processor may be configured to compare the calculated time duration to the received CPR time period and modify the CPR treatment protocol or the received CPR time period based on the comparison. Additionally, the processor may be configured to cause the user interface to provide prompts for implementing the modified CPR treatment protocol over the received CPR period or the CPR treatment protocol over the modified CPR time period.

In another aspect, a method for adjusting a cardiopulmonary resuscitation (CPR) treatment is disclosed. The method may comprise determining a CPR treatment protocol from one or more CPR treatment protocols. The CPR treatment protocol may include a ratio of a number of chest compressions to one or more ventilations for administering during a CPR cycle. The method may also comprise receiving a CPR time period for the CPR treatment, determining a number of CPR cycles for the CPR treatment based on the CPR treatment protocol, and calculating a time duration of the CPR treatment based on the number of CPR cycles of the CPR treatment protocol. Further, the method may comprise comparing the calculated time duration to the received CPR time period and modifying the CPR treatment protocol or the received CPR time period based on the comparison. Additionally, the method may comprise causing the user interface to provide prompts for implementing the modified CPR treatment protocol over the received CPR period or the CPR treatment protocol over the modified CPR time period.

In still another aspect, a non-transitory computer-readable medium storing instructions is disclosed that, when the instructions are executed by one or more processors, causes the one or more processors to perform operations for adjusting a cardiopulmonary resuscitation (CPR) treatment. The operations may comprise determining a CPR treatment protocol from one or more CPR treatment protocols. The CPR treatment protocol may include a ratio of a number of chest compressions to one or more ventilations for administering during a CPR cycle. The operations may also comprise receiving a CPR time period for the CPR treatment, determining a number of CPR cycles for the CPR treatment based on the CPR treatment protocol, and calculating a time duration of the CPR treatment based on the number of CPR cycles of the CPR treatment protocol. Further, the operations may comprise comparing the calculated time duration to the received CPR time period and modifying the CPR treatment protocol or the received CPR time period based on the comparison. Additionally, the operations may comprise causing the user interface to provide prompts for implementing the modified CPR treatment protocol over the received CPR period or the CPR treatment protocol over the modified CPR time period.

In still another aspect, a portable medical device for adjusting a CPR treatment is disclosed. The portable medical device includes a user interface configured to provide prompts to a user. The portable medical device also includes a memory device configured to store one or more CPR treatment protocols. The portable medical device also includes a processor configured to determine a particular CPR treatment protocol from the one or more CPR treatment protocols. The particular CPR treatment protocol indicates a ratio of chest compressions to ventilations for administering during a CPR cycle. The processor is configured to receive an indication of a CPR time period for the CPR treatment. The processor is configured to determine a number of CPR cycles for the CPR treatment based on the particular CPR treatment protocol. The processor is configured to calculate a time duration of the CPR treatment based on the number of CPR cycles of the particular CPR treatment protocol. The processor is configured to compare the time duration to the CPR time period. The processor is configured to modify the particular CPR treatment protocol or the CPR time period based on a comparison between the time duration and the CPR time period. The processor is configured to cause the user interface to provide prompts for (i) implementing a modified version of the particular CPR treatment protocol over the CPR time period or (ii) implementing the particular CPR treatment protocol over a modified CPR time period.

In still another aspect, a method for adjusting a CPR treatment provided by a defibrillator is disclosed. The method includes determining, by one or more processors of the defibrillator, a particular CPR treatment protocol from one or more CPR treatment protocols. The particular CPR treatment protocol indicates a ratio of chest compressions to ventilations for administering during a CPR cycle. The method includes receiving, by the one or more processors, an indication of a CPR time period for the CPR treatment. The method includes determining, by the one or more processors, a number of CPR cycles for the CPR treatment based on the particular CPR treatment protocol. The method includes calculating, by the one or more processors, a time duration of the CPR treatment based on the number of CPR cycles of the particular CPR treatment protocol. The method includes comparing, by the one or more processors, the time duration to the CPR time period. The method includes modifying the particular CPR treatment protocol or the CPR time period based on a comparison between the time duration and the CPR time period. The method includes causing a user interface to provide prompts for (i) implementing a modified version of the particular CPR treatment protocol over the CPR time period or (ii) implementing the particular CPR treatment protocol over a modified CPR time period.

In still another aspect, a non-transitory computer-readable medium is disclosed. The non-transitory computer-readable medium includes instructions that, when executed by a processor of a defibrillator, cause the processor to perform operations for adjusting a CPR treatment. The operations include determining a particular CPR treatment protocol from one or more CPR treatment protocols. The particular CPR treatment protocol indicates a ratio of chest compressions to ventilations for administering during a CPR cycle. The operations include receiving an indication of a CPR time period for the CPR treatment. The operations include determining a number of CPR cycles for the CPR treatment based on the particular CPR treatment protocol. The operations include calculating a time duration of the CPR treatment based on the number of CPR cycles of the particular CPR treatment protocol. The operations include comparing the time duration to the CPR time period. The operations include modifying the particular CPR treatment protocol or the CPR time period based on a comparison between the time duration and the CPR time period. The operations include causing a user interface to provide prompts for (i) implementing a modified version of the particular CPR treatment protocol over the CPR time period or (ii) implementing the particular CPR treatment protocol over a modified CPR time period.

The foregoing summary is illustrative only and is not intended to be in any way limiting. In addition to the illustrative aspects, embodiments, and features described above, further aspects, embodiments, and features will become apparent by reference to the figures and the following detailed description.

### BRIEF DESCRIPTION OF THE DRAWINGS

A more complete understanding of embodiments of the present application may be derived by referring to the detailed description and claims when considered in conjunction with the following figures, wherein like reference numbers may refer to similar elements throughout the figures. The figures are provided to facilitate understanding of the disclosure without limiting the breadth, scope, scale, or applicability of the disclosure. The drawings are not necessarily made to scale.
FIG. 1 is a diagram illustrating a representation of an example scene showing the use of a medical device for monitoring and delivering treatment to a person or patient experiencing a medical condition, in accordance with an example implementation;
FIG. 2 illustrates a front view of a medical device, in accordance with an example implementation;
FIG. 3 illustrates a simplified block diagram of the components of a medical device, in accordance with an example implementation;
FIG. 4 illustrates a graphical user interface of a medical device, in accordance with an example implementation;
FIG. 5 is a table illustrating CPR treatment protocols for various CPR periods, in accordance with an example implementation; and
FIG. 6 is a flowchart illustrating a method of adjusting or modifying CPR treatments implemented by a medical device, in accordance with an example implementation.

### DETAILED DESCRIPTION

The figures and the following description illustrate specific exemplary embodiments. It will be appreciated that those skilled in the art will be able to devise various arrangements that, although not explicitly described or shown herein, embody the principles described herein and are included within the scope of the claims that follow this description. Furthermore, any examples described herein are intended to aid in understanding the principles of the disclosure and are to be construed as being without limitation. As a result, this disclosure is not limited to the specific embodiments or examples described below, but by the claims and their equivalents.

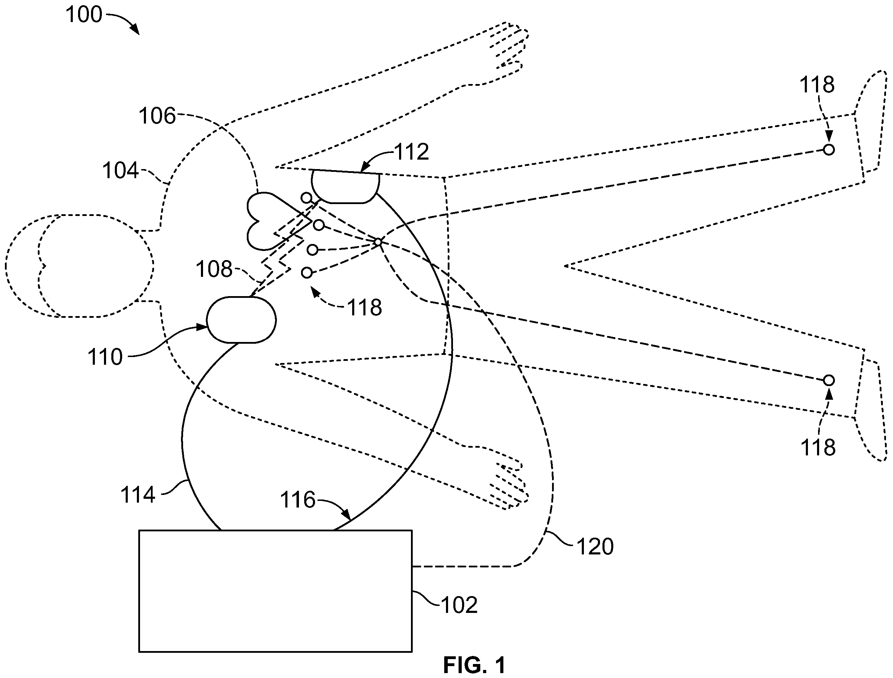

Particular embodiments are described herein with reference to the drawings. In the description, common features may be designated by common reference numbers throughout the drawings. In some drawings, multiple instances of a particular type of feature may be used. Although these features are physically and/or logically distinct, the same reference number is used for each, and the different instances are distinguished by addition of a letter to the reference number. When the features as a group or a type are referred to herein (e.g., when no particular one of the features is being referenced), the reference number is used without a distinguishing letter. However, when one particular feature of multiple features of the same type is referred to herein, the reference number is used with the distinguishing letter. For example, referring to Figure 1, sensors are illustrated and associated with reference number 118. When referring to a particular one of the sensors, such as the sensor 118A, the distinguishing letter "A" may be used. However, when referring to any arbitrary one of the sensor or to the sensors as a group, the reference number 118 may be used without a distinguishing letter.

As used herein, various terminology is used for the purpose of describing particular implementations only and is not intended to be limiting. For example, the singular forms "a," "an," and "the" are intended to include the plural forms as well, unless the context clearly indicates otherwise. Further, the terms "comprise," "comprises," and "comprising" are used interchangeably with "include," "includes," or "including." Additionally, the term "wherein" is used interchangeably with the term "where." As used herein, "exemplary" indicates an example, an implementation, and/or an aspect, and should not be construed as limiting or as indicating a preference or a preferred implementation. As used herein, an ordinal term (e.g., "first," "second," "third," etc.) used to modify an element, such as a structure, a component, an operation, etc., does not by itself indicate any priority or order of the element with respect to another element, but rather merely distinguishes the element from another element having a same name (but for use of the ordinal term). As used herein, the term "set" refers to a grouping of one or more elements, and the term "plurality" refers to multiple elements.

The present application is directed to embodiments that relate to systems, methods, and apparatus for providing treatments to a person or patient experiencing a medical condition, such as cardiac arrest or any other cardiac rhythm abnormality. During an emergency episode, the embodiments may provide instructions to a user or rescuer for performing cardiac or resuscitation treatments, such as CPR, on the person. The embodiments may also be configured to deliver a defibrillation pulse or shock to the person to restore a normal heartbeat.

The embodiments may allow a user or rescuer to input or select information for a cardiac or cardiopulmonary resuscitation (CPR) treatment. The embodiments may also allow a user or rescuer to request or specify a CPR time period for a CPR treatment. Based on the user inputs, the embodiments may be configured to identify and select a CPR treatment protocol from one or more CPR treatment protocols. The CPR treatment protocol selected by the embodiments may include a pattern or ratio of a number of chest compressions to one or more ventilations for a CPR cycle (e.g., a number of chest compressions followed by one or more ventilations during a CPR cycle). The CPR treatment protocol may also include the rate at which the chest compressions should be delivered (e.g., compressions per minute) and the time over which the ventilations should be delivered or provided (e.g., each ventilation should have duration of about one second). The CPR treatment protocol may be consistent with guidelines provided by the American Heart Association or medical directors.

The embodiments may evaluate the selected CPR treatment protocol for the CPR treatment. Based on the evaluation, the embodiments may modify the selected CPR treatment protocol and/or modify the CPR time period inputted or selected by the user for the CPR treatment. For example, the embodiments may change the number of chest compressions for a CPR cycle, change the number of the ventilations for a CPR cycle, adjust a duration or time period of a ventilation, adjust the length or duration of a time period between ventilations, or adjust or modify the CPR time period inputted or selected by the user for the CPR treatment. The embodiments may adjust the CPR treatment protocol while remaining consistent and compliant with current medical guidelines and standards. In some implementations, the processing unit may modify the CPR treatment protocol by changing a number of chest compressions, a time between chest compressions, or a start time for chest compressions for at least one of the CPR cycles in order to provide an integer number of chest compressions for a CPR time period. Thus, the processing unit 350 may override or modify the specific CPR time periods input by a user or rescuer when those inputs are inconsistent with the CPR protocol so that the adjusted or modified CPR treatment protocol provides a best fit for the user inputs (such as an integer number of chest compressions with a CPR time period), satisfies the overall user requirements, and remains complaint and consistent with the guidelines of the AHA. Based on the modified or modified CPR treatment protocol and/or the modified CPR time period inputted or selected by the user, the embodiments may provide rhythmic signals to guide a user in pacing and timing of chest compressions and may also provide signals to guide in the pacing and timing of ventilations for the CPR treatment.

Referring now to the figures, Figure 1 is a diagram of a representation of an exemplary scene 100 showing use of a medical device 102 for monitoring and providing treatment or therapy to a person 104 experiencing a medical condition, such as cardiac arrest. The medical device 102 may be operated by a user (e.g., a healthcare professional, service worker, a doctor, a first responder, etc.) and may be used in a hospital or a pre-hospital environment or setting. The medical device 102 may include functions and operations of an external defibrillator, such as a manual defibrillator, an automatic external defibrillator (AED), or any other suitable defibrillator. In some examples, the medical device 102 may be a monitor defibrillator, which is a combination of a monitor and a defibrillator.

As shown in Figure 1, the medical device 102 is positioned near the person 104 (e.g. a patient). The person 104 may be experiencing a condition in his or her heart 106, which could be, for example, cardiac arrest or any other cardiac rhythm abnormality. The person 104 may be lying on his or her back on a surface, such as the ground or a bed, and may be located in a hospital, a home, or a pre-hospital environment (e.g., an ambulance). The medical device 102 may be configured to generate an electrical pulse 108 and deliver the electrical pulse 108 to the person 104. The electrical pulse 108, also known as a defibrillation shock or therapy shock, is intended to go through and restart the heart 106, in an effort to save the life of the person 104. The electrical pulse 108 can further include one or more pacing pulses and the like.

The electrical pulse 108 may be delivered to the person 104 using defibrillation electrodes 110 and 112. The defibrillation electrodes 110 and 112 may include hand-held electrode paddles or electrode pads placed on the body of the person 104 (e.g., the chest of the person 104). An electrical cable 114 may connect the defibrillation electrode 110 to the medical device 102 and an electrical cable 116 may connection the defibrillation electrode 112 to the medical device 102. When the defibrillation electrodes 110 and 112 are in electrical contact with the body of person 104, the medical device 102 may administer, via the defibrillation electrodes 110 and 112, the electrical pulse 108 through the heart 106 of the person 104. The defibrillation electrodes 110 and 112 may also be configured to sense or detect one or more physiological parameters of the person 104 and to generate signals representative of the physiological parameters.

As shown in phantom in FIG. 1, one or more sensors or electrodes 118 may be placed at various locations on the body of the person 104. The sensors 118 may be configured to sense or detect physiological parameters of the person 104 and to produce signals representative of the physiological parameters. An electrical cable 120 may connect the sensors 118 to the medical device 102. The physiological parameters generated by the sensors 118 and/or the defibrillation electrodes 110 and 112 may be provided to the medical device 102 for evaluation and analysis. The physiological parameters may include ECG data, heart rhythm data, heart rate data, cardiac output data, blood flow data, a level of perfusion, respiration data, body temperature data, and/or any other suitable physiological parameter that may be used to assess the physical condition of the person 104.

The medical device 102 may include a user interface having an input device configured to receive input or selections from a user or rescuer for determining a cardiac or resuscitation treatment (e.g., a CPR treatment) for the person 104. The user interface may allow the user to input or select an age classification of the person 104 (e.g., adult or child/infant), an airway status (e.g., whether the person 104 has his or her airway secured by artificial airway), whether the CPR is being delivered by one or two persons, whether the person delivering CPR is a medical professional or a layperson, and/or other variables or factors which may be used by the medical device 102 to determine or select a CPR treatment protocol for the medical treatment of the person 104. In some implementations, the medical device 102 may be automatically configured, upon activation or set-up, for an adult with an unsecured airway. The user interface of the medical device 102 may also allow a user to request or specify a time period for a cardiac or resuscitation treatment. For example, the user may input or select a CPR time period for performing a CPR treatment. In some implementations, the user may input or select a CPR time period from one or more preset or predetermined CPR periods (e.g., 15 seconds, 1 minute, 2 minutes, 3 minutes, etc.).

The medical device 102 may be configured to select a CPR treatment protocol for delivery of a CPR treatment to the person 104 based on the user inputs and/or physiological parameters of the person 104. For example, the medical device 102 may identify and retrieve a CPR treatment protocol from memory based on the inputs from a user. The CPR treatment protocol may include a pattern or ratio of a number of chest compressions to one or more ventilations for a CPR cycle of the CPR treatment (e.g., a number of chest compressions to be delivered followed by one or more ventilations during a CPR cycle). The medical device 102 may modify or adjust the selected CPR treatment protocol or the CPR time period inputted or selected by a user for the CPR treatment as further described below.

The user interface of the medical device 102 may also include output devices to provide visual or aural signals to the user. For example, the output devices may provide signals and prompts to assist a user or rescuer in delivering a cardiac or resuscitation treatment, such as a CPR treatment, to the person 104. The CPR treatment may be based on the CPR treatment protocol and/or the modified or adjusted CPR treatment protocol. The output devices of the user interface may provide rhythmic signals to guide a user in pacing and timing of chest compressions of the CPR treatment and may provide signals to guide in the pacing and timing of ventilations of the CPR treatment. Further, the output devices may display representations of physiological parameters to assist a user in treating and diagnosing medical conditions of the person 104. Additionally, the output devices may provide instructions to a user for delivering a defibrillation pulse to the person 104.

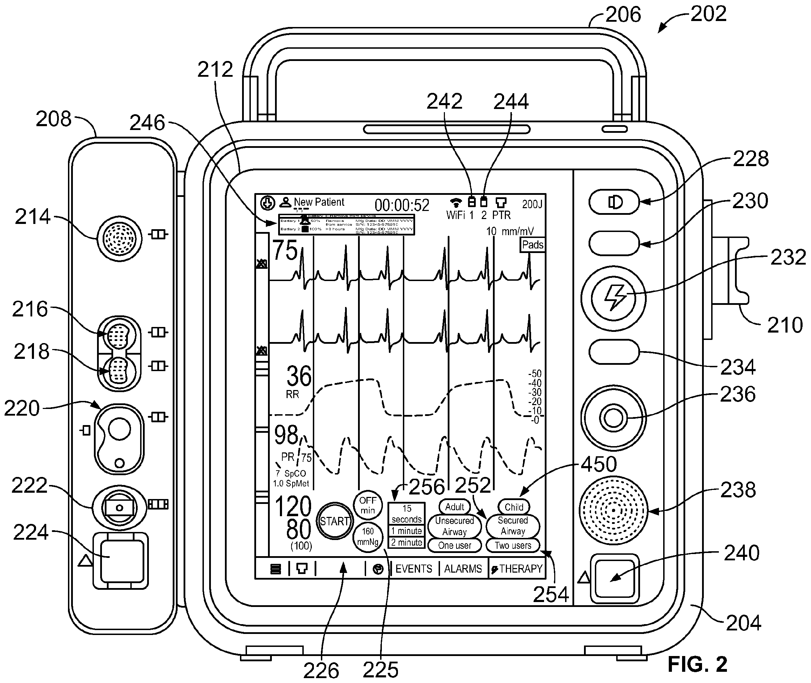

Figure 2 illustrates a front view of a medical device 202, in accordance with an example implementation. The medical device 202 can comprise the medical device 102 of Figure 1. The medical device 202 may be configured to monitor and provide treatment or care to a person or patient experiencing a medical condition, such as cardiac arrest or any other cardiac rhythm abnormality. The medical device 202 may be operated by a user (e.g., a medical professional, a first responder, a healthcare professional, service worker, a doctor, etc.) and may be used in a hospital or a pre-hospital environment or setting. As illustrated, the medical device 202 may be a monitor defibrillator, which is a combination of a monitor and a defibrillator.

As a defibrillator, the medical device 202 may be configured to deliver an electrical pulse or shock to a person experiencing a medical condition. The medical device 202 may be configured to operate or function in one or more defibrillation modes, such as a manual defibrillation mode, an AED mode, or any other suitable defibrillation mode. For example, the medical device 202 may be selected to operate in an AED mode when the medical device 202 is intended to be used by a first responder and/or a layperson who may have no or minimal training in providing medical treatment using defibrillation. When operating in the AED mode, the medical device 202 may determine whether a defibrillation pulse or shock is needed and, if so, charge an energy storage device (e.g., a capacitor) of the medical device 202 to a predetermined energy level and instruct the user to administer the defibrillation shock. The medical device 202 may also be selected to operate in a manual defibrillation mode when the medical device 202 is intended to be used by persons who are trained to provide medical treatment using defibrillation (e.g., medical professionals, such as doctors, nurses, paramedics, emergency medical technicians, etc.). When the medical device 202 is configured to operate in the manual defibrillation mode, the user may determine whether a defibrillation pulse or shock is needed and may control the administration of the defibrillation shock to the person.

As a monitor, the medical device 202 may monitor and evaluate physiological parameters of a person being treated for a medical condition. The medical device 202 may receive signals or voltages from one or more electrodes or sensors placed at various locations on the body of the person. The electrodes may sense or detect the physiological parameters of the person and produce signals representative of the physiological parameters. The representations of the physiological parameters may be displayed by the medical device 202 to assist a user in treating and diagnosing medical conditions of a person. The physiological parameters may include ECG (electrocardiogram) data, body temperature, non-invasive blood pressure (NIBP), arterial oxygen saturation/pulse oximetry (SpO2), a concentration or partial pressure of carbon dioxide in the respiratory gases (e.g., capnography), and/or any other suitable physiological parameter of a person. The physiological parameters of the person can be stored in the memory of the medical device 202. In some examples, the physiological parameters may be transmitted to other remote communication or computing devices and databases.

As shown in Figure 2, the medical device 202 includes a housing or casing 204, a handle 206, an input module or interface 208, a defibrillation interface 210, and a user interface 212. The handle 206 of the medical device 202 is attached to the housing 204 to allow a user to move or transport the medical device 202 to a location near a person experiencing a medical condition. The housing 204 may be generally rectangular or square shaped. In other examples, the housing 204 can have any other suitable shape. The housing 204 may be formed from various materials or combinations of materials. For example, the housing 204 may be made of molded plastic, metal, or some combination of both.

The input module 208 of the medical device 202 may be coupled to or integral with the housing 204. The input module 208 may enable the medical device 202 to receive physiological parameters (e.g., a heart rate (HR) and ECG data) of a person via sensors (e.g., the sensors 118 of Figure 1). The sensors may be placed on the body of a person to sense or detect signals generated by the body or heart of the person. The input module 208 may include one or more ports or receptacles to enable the sensors to be detachably coupled to the input module 208. As shown in Figure 2, the input module 208 can include a port 214 configured to be connected to an oxygen saturation (SpO2) sensor, a port 216 configured to be connected to a temperature sensor, a port 218 configured to be connected to a sensor for measuring invasive blood pressure (IP) via a catheter, a port 220 configured to be connected to a sensor for measuring partial pressure of carbon dioxide (CO2) in gases in the airway via capnography, and a port 222 configured to be connected to a sensor for measuring a non-invasive blood pressure (NIBP). The input module 208 may also include a communication port 224, such as a Universal Serial Bus (USB) port, that can be used to connect to an input device (e.g., a mouse, a keyboard, etc.). The input module 208 may include other ports to enable any other suitable physiologic parameter of a person to be monitored and evaluated by the medical device 202.

The defibrillation interface 210 of the medical device 202 may be configured to enable electrical charges or pulses to be delivered or applied to a person via defibrillation electrodes (e.g., the defibrillation electrodes 110 and 112 shown in Figure 1). The defibrillation electrodes may also be configured to enable the medical device 202 to receive physiological parameters of a person (e.g., a heart rate (HR), ECG data, etc.). The defibrillation interface 210 may include one or more ports or receptacles capable of allowing the defibrillation electrodes to be detachably coupled to the defibrillation interface 210. A cable assembly or therapy cable may enable the defibrillation electrodes to be coupled to the ports of the defibrillation interface 210. Each therapy cable may include a defibrillation electrode attached at one end and a connector attached to the other end. The connector may be configured to be coupled to or plugged into a port or receptacle of the defibrillation interface 210.

The user interface 212 of the medical device 202 may include one or more input devices configured to receive inputs or commands from a user and one or more output devices configured to provide information to the user. The input devices of the user interface may include various controls, such as switches, pushbuttons, keyboards, touchscreens, microphones, scanners, and/or cameras, etc., for operating the medical device 202. The output devices of the user interface 212 can be visual, audible or tactile, for communicating to a user of the medical device 202 (e.g., a medical professional, a first responder, etc.). For example, the output devices may be configured to present visual alarms or alerts, flashing lights, and/or warnings to the user. The output devices may also include an audio system that provides audio signals to aurally communicate with the user voice prompts that deliver instructions or commands, monotonal, ascending, descending or quickening tones to indicate alerts or warnings, and/or any other suitable audio signals for communicating with the user of the medical device 202.

As shown in Figure 2, the user interface 212 of the medical device 202 includes a power button 228 configured to turn the medical device 202 on and off (e.g., "On-Off" button), a charge button 230 configured to cause the medical device 202 to build an electric charge to be applied to the person in a form of a defibrillation shock or pulse, a defibrillation shock button 232 configured to cause the medical device 202 to apply a defibrillation pulse or therapy shock to a person during a defibrillation episode, an analyze button 234 configured to cause the medical device 202 to analyze physiologic parameters of a person (e.g., ECG data) to facilitate determining the appropriate time to apply a defibrillation pulse or shock, and a speed dial button 236 configured to navigate through menus of on-screen software. The user interface 212 may also include a speaker 238 to provide audio output and a USB output port 240 to facilitate connecting the medical device 202 to a device, such as a printer. The user interface 212 may include any other suitable output device for providing outputs or input device for receiving inputs from a user.

The user interface 212 of the medical device 202 may also include a display device 225 (e.g., a touchscreen) for displaying a graphical user interface (GLTI) 226. The GUI 226 may display physiologic parameters of a person (e.g., a patient), provide visual feedback to the user (e.g., a healthcare provider) about a condition of the person, provide information about the medical device 202 (e.g., battery information), and allow the user to interact with and operate the medical device 202. The GUI 226 may also be configured to visually present various measured or calculated parameters associated with the person indicating the physical status of the person (e.g., an ECG). Further, the GUI 226 may provide instructions and/or commands, including prompts to perform cardiopulmonary resuscitation (CPR) treatment or other treatment, to the user. Additionally, the GUI 226 may include multiple visual user interface items that are selectable or "clickable" by the user including user-selectable icons, user-selectable on-screen buttons, menus, widgets, scroll bars, and graphical objects, and/or other items for facilitating user interaction.

As shown in Figure 2, the GUI 226 includes graphical representations of a first battery unit indicator 242 and a second battery unit indicator 244. The first battery unit indicator 242 and second battery unit indicator 244 are configured to display a status of a first battery unit and a status of a second battery unit, respectively, of the medical device 202. For example, the first and second battery unit indicators 242 and 244 may provide a charging level of the first and second battery units. The GUI 226 may also display messages 246 and information to the user or rescuer.

The GUI 226 may also allow a user or rescuer to input information (e.g., parameters, variables, etc.) for enabling the medical device 202 to identify and select a cardiac or resuscitation treatment protocol (e.g., a CPR treatment protocol) for a person experiencing a medical condition, such as cardiac arrest. For example, the GUI 226 may allow the user to input or select an age classification of a person (e.g., adult or child/infant), an airway status (e.g., whether the person has his or her airway secured by artificial airway), whether the CPR is being delivered by one or two persons, whether the person delivering CPR is a medical professional or a layperson, and/or other variables which may be used by the medical device to determine or select a CPR treatment protocol. The GUI 226 may enable a user to input the information through user-selectable on-screen graphical items. In some implementations, the user interface may include the following user-selectable user-interface items: "Adult" and "infant/child" 250, "secured airway" and "unsecured airway" 252, and "one provider" and "two providers" 254. The user-selectable user-interface items may allow the user to provide input by pressing or selecting areas on the GUI 226 displaying the items. In some implementations, the medical device 202 may be automatically configured, upon activation or set-up, for an adult with an unsecured airway.

The GUI 226 of the medical device 202 may also allow a user to input a time period for performing a cardiac or resuscitation treatment, such as a CPR time period for performing a CPR treatment. For example, the GUI 226 may allow a user to input or select a CPR time period for performing a CPR treatment from preset or predetermined time limits. The GUI 226 may display user-selectable user-interface items 256 to allow a user to input or select the CPR time periods. In some implementation, the GUI 226 may include the following user-selectable user-interface items: "15 seconds", "1 minute, "2 minutes", and "3 minutes".

Once the user inputs or selects a CPR time period for a CPR treatment, the medical device 202 may determine or select, based on the user inputs or selections, a CPR treatment protocol for the CPR treatment from memory. The medical device 202 may modify the CPR treatment protocol and/or modify the CPR time period inputted or selected by the user for the CPR treatment as further described below. The GUI 226 may provide signals and prompts to assist a user or rescuer for delivering the CPR treatment according to the modified CPR treatment protocol or the modified or adjusted CPR time period. For example, the GUI 226 of the medical device 202 may provide rhythmic signals to guide a user in pacing and timing of chest compressions for the modified CPR treatment and may also provide signals to guide in the pacing and timing of ventilations for the modified CPR treatment.

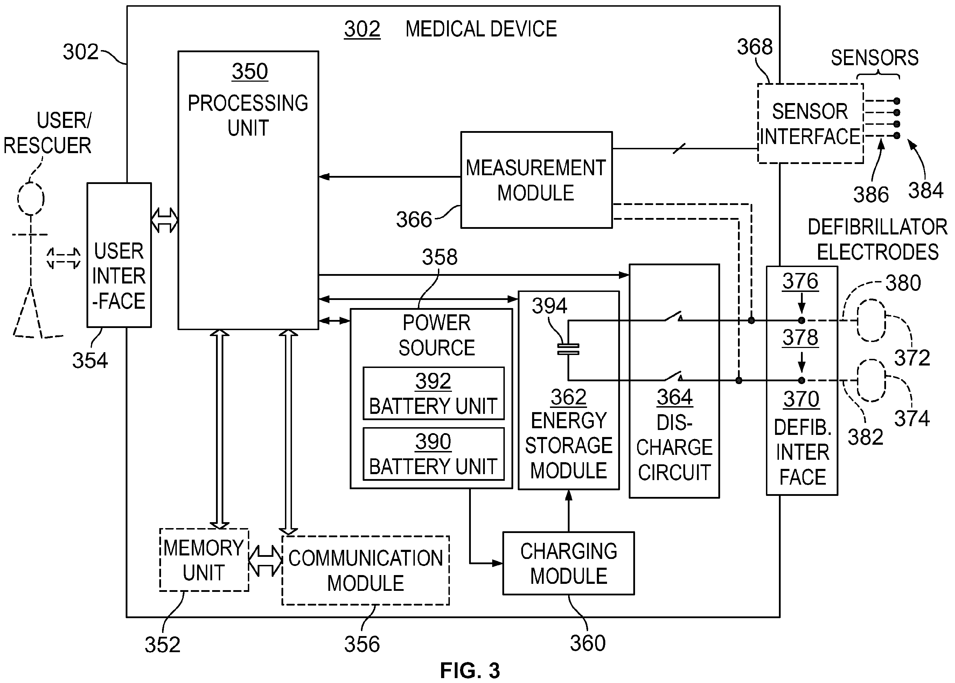

FIG. 3 is a diagram showing components of a medical device 302, in accordance with an exemplary embodiment. As shown in Figure 3, the medical device 302 includes a processing unit 350, a memory unit 352, a user interface 354, a communication module or interface 356, a power source 358, a charging module 360, an energy storage module or device 362, a discharge circuit 364, a measurement module 366, a sensor interface 368, and a defibrillator interface 370. These components can be, for example, included in the medical devices of Figures 1 or 2.

The defibrillation interface 370 of the medical device 302 may be configured to enable an electrical pulse or shock to be delivered or applied to a person (e.g., a patient) experiencing a medical condition. The defibrillation interface 370 may comprise the defibrillation interface 210 of Figure 2. The defibrillation interface 370 may include one or more ports or nodes 376 and 378 to enable the defibrillation electrodes 372 and 374 to be detachably coupled to the defibrillation interface 370. A cable assembly or therapy cable 380 may enable the defibrillation electrode 372 to be coupled to the port 376 of the defibrillation interface 370 and a cable assembly or therapy cable 382 may enable the defibrillation electrode 374 to be coupled to the port 378 of the defibrillation interface 370 of the medical device 302. The cable assembly 380 may include the defibrillation electrode 372 attached at one end and a connector attached to the other end, and the cable assembly 380 may include the defibrillation electrode 374 attached at one end and a connector attached to the other end. The connectors of the cable assemblies 380 and 382 may be configured to be coupled to or plugged into the ports 376 and 378 of the defibrillation interface 370. The connectors can be male or female connectors that are compatible with the ports 376 and 378 of the defibrillation interface 370 of the medical device 302.

The defibrillation interface 370 may also enable the medical device 302 to receive physiological parameters (e.g., a heart rate (HR) and ECG data) of the person or patient from the defibrillation electrodes 372 and 374. Each of the defibrillation electrodes 372 and 374 may be configured to measure one or more physiological parameters of the person (e.g., heart electrical activity, heart rate, etc.) and to provide signals representative of the physiological parameters to the defibrillation interface 370. For example, when the defibrillation electrodes 372 and 374 are placed on the chest of the person, an ECG signal of the person can be detected as a voltage difference between the defibrillation electrodes 372 and 374. The defibrillation electrodes 372 and 374 may also be used to determine device parameters indicative of a condition of the medical device 302, such as an electrode impedance or a user interaction with the medical device 302. For example, the medical device 302 can detect an impedance between the defibrillation electrodes 372 and 374 to determine whether the defibrillation electrodes 372 and 374 are making sufficient electrical contact with a person's body.

The sensor interface 368 of the medical device 302 may be configured to receive physiological parameters (e.g., a heart rate (HR) and ECG data) from one or more sensors 384 (e.g., physiological sensors). The sensors 384 may be placed in contact with the body of a person being monitored or treated for a medical condition. Each of the sensors 384 may include a transducer configured to sense a signal or voltage of the person. For example, the sensors 384 may measure or detect heart electrical activity and other parameters, such as an electrocardiogram (ECG), saturation of the hemoglobin in arterial blood with oxygen (SpO2), carbon monoxide (carboxyhemoglobin, COHb) and/or methemoglobin (SpMet), partial pressure of carbon dioxide (CO2) in gases in the airway by means of capnography, total air pressure in the airway, flow rate or volume of air moving in and out of the airway, blood flow, blood pressure (e.g., non-invasive blood pressure (NIBP)), core body temperature with a temperature probe in the esophagus, oxygenation of hemoglobin within a volume of tissue (rSO2), and any other physiological parameters of a person being monitored or treated.

The sensor interface 368 may include one or more receptacles or ports (e.g., an ECG port) to enable the sensors 384 to be detachably coupled to the medical device 302. The sensors 384 may be attached to the sensor interface 368 by cable assembles or therapy cables 386. In some embodiments, the sensors 384 may be fixedly connected to the sensor interface 368. The therapy cables 386 may each include a sensor 384 at one end and a connector at the opposite end. The connector can be configured to be coupled to or plugged into a port or receptacle of the sensor interface 368.

The measurement module 366 of the medical device 302 may be configured to receive signals or sensor data from the sensor interface 368 and the defibrillation interface 370. The signals may be representative of physiological parameters or conditions of a person being monitored and/or treated for a medical condition. The measurement module 366 may measure or determine various physiological parameters from the signals. For example, the measurement module 366 may determine an ECG of the person based on a voltage difference between the defibrillation electrodes 372 and 374. The measurement module 366 may also determine device parameters indicative of a condition of the medical device 302, such as an electrode impedance or a user interaction with the medical device 302. For example, the measurement module 366 may measure an impedance or voltage across the defibrillation electrodes 372 and 374 or a pair of sensors 384.

The measurement module 366 may also include a filter circuit or hardware (e.g., amplifiers, filters, etc.) to attenuate and/or filter at least some of the noise that may be present on the signals received from the sensor interface 368 and/or the defibrillation interface 370. For example, the filter circuit may apply at least one filter to the signal to remove artifacts resulting from chest compressions being delivered to the person. In some embodiments, the filter may be implemented as an analog filter, a digital filter, or combinations of both. The measurement module 366 may also digitize the signals received from the sensor interface 368 and/or defibrillation interface 370 prior to transmitting the signals to the processing unit 350.

The user interface 354 of the medical device 302 may facilitate user interactions with the medical device 302. The user interface 354 may comprise the GUI 226 of Figure 2. The user interface 354 may include various types of input devices for receiving inputs or commands from a user. For example, the input devices may include keyboards, switches, microphones, pushbuttons, touchscreens, scanners, and/or any other suitable input device for enabling inputs to the medical device 302. For instance, a user can use the input devices on the user interface 354 to input information regarding a particular event (e.g., a treatment or medication administered to the person).

The input devices of the user interface 354 may also allow a user or rescuer to input information (e.g., parameters, variables, etc.) for enabling the medical device 302 to identify and select a cardiac or resuscitation treatment (e.g., a CPR treatment) for a person experiencing a medical condition, such as cardiac arrest. For example, the user devices of the user interface 354 may allow the user to input or select an age classification of a person (e.g., adult or child/infant), an airway status (e.g., whether the person has his or her airway secured by artificial airway), whether the CPR is being delivered by one or two persons, whether the person delivering CPR is a medical professional or a layperson, and/or other variables which may be used by the medical device 102 to select and/or deliver a CPR treatment protocol. In some implementations, the medical device 302 may be automatically configured, upon activation or set-up, for an adult with an unsecured airway.

The input devices of the user interface 354 may also allow the user to input or select a time period for performing a cardiac or resuscitation treatment, such as a CPR time period for performing a CPR treatment. In some implementations, the user may select or input a CPR period for the CPR treatment from preset or predetermined time limits (e.g., 15 seconds, 1 minute, 2 minutes, 3 minutes, etc.). Once the user inputs or selects the CPR time period for the CPR treatment, the medical device 302 may determine or select, based on the user inputs, a CPR treatment protocol from memory (e.g., the memory unit 352). The medical device 302 may evaluate the CPR treatment protocol for the CPR treatment. Based on the evaluation, the medical device 302 may modify the CPR treatment protocol and/or may modify or adjust the CPR time period inputted or selected by the user for the CPR treatment as further described below.

The user interface 354 may also comprise various types of output devices for providing information to the user. For example, the output devices may provide instructions or prompts to assist a user or rescuer for delivering CPR treatments to a person experiencing a medical condition, such as cardiac arrest. The output devices of the user interface can be visual, audible or tactile for communicating to or providing feedback to a user (e.g. a rescuer, a first responder, a healthcare professional, etc.). The output devices may include a screen, a display, one or more light emitting diodes (LEDs), and/or a speaker to output various sounds (e.g., voice or audio), etc. The output devices may be configured to present visual alarms or alerts, flashing lights, and/or warnings to the user of the medical device 302. The output devices may also include an audio system that provides an audio signal to aurally communicate with user voice prompts that deliver instructions or commands, monotonal, ascending, descending or quickening tones to indicate alerts or warnings, and/or any other suitable output device for communicating with the user.

Further, the output devices of the user interface 354 may include a metronome or a metronome system for providing signals to guide the user in pacing and timing of chest compressions for cardiac or resuscitation treatments (e.g., a CPR treatment) and, in some implementations, for providing signals to guide in the pacing and timing of ventilations for the a cardiac or resuscitation treatment. The signals may be visual (such as flashing lights or graphics on a display screen), or may he aural. In some implementations, the metronome may deliver to the user a first type of signal for chest compressions, a second type of signal for ventilations, and a third type of signals to indicate an upcoming transition from compressions to ventilations (or, in a protocol where ventilations are given without a pause in compressions, to indicate an upcoming ventilation series). The different signal types may be distinguishable from one another by the user. When flashing lights are used, different colors may distinguish between compressions, transitions, and ventilations. The aural signals may be any of a variety of sounds such as tones, beeps, tocks, clicks, and the like, or may be voiced (for example, "press-press-press" for compressions, "ventilate" or "blow" for ventilations). In some embodiments, a user may choose whether to have the metronome deliver voiced or non-voiced sounds (e.g., tones, beeps, clicks, tocks, or other non-verbal sounds) through a set-up menu upon device set-up.

The signals for chest compressions may be rhythmic signals such as a series of identical sounds delivered at a rate corresponding to the desired rate for chest compressions. A sound that is suggestive of or approximates the sound of ventilation may be used for a ventilation signal. The sound signal used for each ventilation may have a duration that corresponds to the desired duration of the ventilation. For a ventilation series having more than one ventilation, the ventilation sound signals may also be delivered at a rate equal to the desired rate for ventilation delivery. The transition signals may advise the user that a transition from chest compressions to ventilations (with or without a pause in chest compressions) is coming up soon. For example, where tones, beeps, clicks or tocks are used to indicate chest compressions for a cardiac or resuscitation treatment (e.g., 30:2 compressions to ventilations protocol for a CPR treatment protocol), the transition signal may be a voiced countdown of the last few compressions in a series.

Further, the output devices of the user interface 354 may include a display configured to visually present to the user various measured or calculated parameters associated with a person or patient experiencing a medical condition, such as cardiac arrest. For example, the display can be configured to visually present an ECG and/or other physiological signals indicating the physical status of the person in cardiac arrest. The output devices may also provide instructions and/or commands, including prompts to perform CPR treatments or other treatments, to the user.

The memory unit 352 of the medical device 302 may be in operable communication with the processing unit 350. The memory unit 352 may store various values, look-up tables, equations, audio and video files, and/or one or more CPR treatment protocols that can be read and accessed by the processing unit 350. The CPR treatment protocols may vary depending on factors such as age classification of the person experiencing a medical condition (e.g., adult or child/infant), an airway status (e.g., whether the person has his or her airway secured by artificial airway), whether the CPR is being delivered by one or two persons, or whether the person delivering CPR is a medical professional or a layperson. In some implementations, the CPR treatment protocol may be the same or similar when the CPR treatment is delivered by 1 person or 2 persons. Each of the CPR treatment protocols may include a rate of chest compressions, a number of chest compressions in a sequence or the time duration of a sequence of chest compressions, the frequency and rate of ventilations, a number of ventilations, and a time duration of each single ventilation. The CPR treatment protocols may also include instructions for the delivery of chest compressions, ventilations, and/or defibrillator pulses.

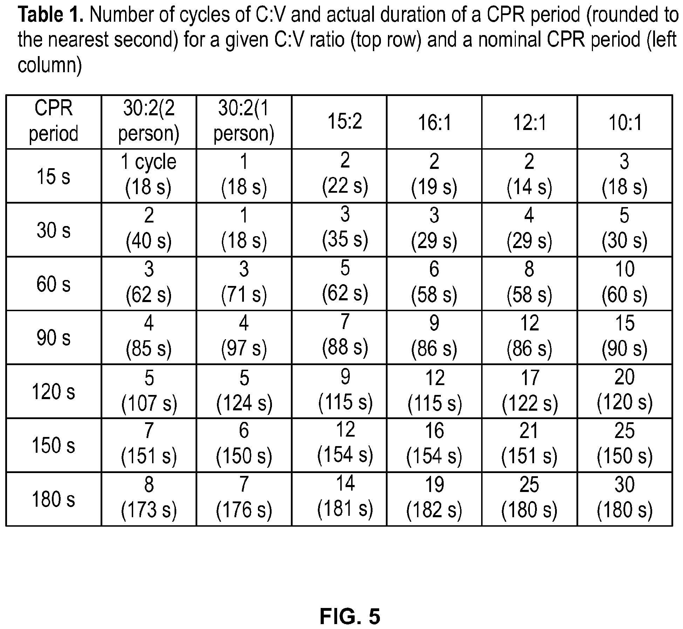

The CPR treatment protocols may be compliant and consistent with the guidelines from the American Heart Association (AHA) and medical directors. For example, the AHA guidelines may recommend that with an adult and a non-secured airway rescuers perform CPR using chest compressions and ventilation (e.g., mouth-to-mouth breathing) at a ratio of 30:2 compressions-to-breaths. The AHA Guidelines may also recommend 5 cycles in a CPR treatment, with only compressions in the last cycle. Accordingly, the CPR treatment protocols may be stored in the memory unit 352 of the medical device 302 according to the AHA guidelines. Figure 5 shows a table of the CPR treatment protocols for an adult with an unsecure airway for various CPR periods (e.g., CPR time periods) that may be stored in the memory unit 352 and used by the medical device 302 to select and determine a CPR treatment protocol. The memory unit 352 can also store a person's sensed physiological parameters, historical data, and defibrillation pulses previously discharged.

Further, the memory unit 352 can store instructions or computer executable code of software routines that can be retrieved and executed by the processing unit 350. The memory unit 352 may include one or more computer-readable storage media that can be read or accessed by the processing unit 350. The computer-readable storage media can include volatile and/or non-volatile memory, dynamic random-access memory (DRAM) read-only memory (ROM), random access memory (RAM), magnetic disk storage media, optical storage media, smart cards, flash memory devices, or any other suitable memory. In some embodiments, the computer-readable storage media can be integrated in whole or in part with the processing unit 350. The computer-readable storage media may be implemented using a single physical device (e.g., one optical, magnetic, organic or other memory or disc storage unit), while in other examples, the computer readable storage media can be implemented using two or more physical devices.

The processing unit 350 of the medical device 302 may be configured to control various operations of the medical device 302. The processing unit 350 may receive inputs from the user and various components of the medical device 302 and may process the inputs to produce outputs that may be stored in the memory unit 352 and/or displayed or communicated via the user interface 354. For example, the processing unit 350 may be configured to receive inputs or selections from a user about cardiac or resuscitation treatments (e.g., CPR treatments), identify and retrieve a resuscitation treatment protocol (e.g., a CPR treatment protocol) from memory based on the received inputs or selections of the user, adjust the resuscitation treatment protocol for a resuscitation treatment or an adjusted received resuscitation time period for the resuscitation treatment, and cause the user interface to provide visual or aural signals and prompts to assist a user or rescuer for providing the resuscitation treatment based on the adjusted resuscitation treatment protocol or the adjusted resuscitation time period.

The processing unit 350 of the medical device 302 may receive inputs or selections from the user or rescuer for a CPR treatment. For example, the processing unit 350 may receive information relating to an age classification of a person experiencing a medical condition (e.g., adult or child/infant), an airway status (e.g., whether the person has his or her airway secured by artificial airway), whether the CPR is being delivered by one or two persons, whether the person delivering CPR is a medical professional or a layperson, and/or other variables which may be used by the medical device 102 to determine or select a CPR treatment protocol. The processing unit 350 may also receive information relating to a CPR time period for performing the CPR treatment (e.g., 15 seconds, 1 minute or 60 seconds, 2 minutes or 120 seconds, 3 minutes or 180 seconds, etc.). For example, the processing unit may receive a 120-second CPR time period inputted or selected by a user of the medical device 302. Based on the inputs and/or selections of a user, the processing unit 350 may determine or select a CPR treatment protocol for a CPR treatment from one or more CPR treatment protocols stored in the memory unit 352. For example, the processing unit 350 may select a CPR treatment protocol based on table 1 shown on Figure 5. The memory unit 352 may store the CPR treatment protocol information shown in table 1 of Figure 5. The selected CPR treatment protocol may include a pattern or ratio of chest compressions to ventilations (C:V ratio). The processing unit 350 may also determine a number of cycles of the chest compressions and ventilations to be performed. In some implementations, the processing unit 350 may be configured to select an integer number of CPR cycles of chest compressions and ventilations to be performed during the CPR period inputted or selected by the user for the CPR treatment. For example, the processing unit 350 may determine from tables or information stored in the memory unit (e.g., table 1 of Figure 5) that, for a 120 second CPR time period, the CPR treatment protocol includes five (5) CPR cycles of a 30:2 C:V ratio for 2-rescuers. The processing unit 350 may also determine that, based on table 1 of Figure 5, for a 120 second CPR time period, the CPR treatment protocol includes five (5) CPR cycles of a 30:2 C:V ratio for 1-rescuer.

Once the processing unit 350 determines the number of cycles of chest compressions and ventilations for the CPR treatment, the processing unit 350 may calculate the actual time to complete or perform the CPR treatment protocol. The processing unit 350 may calculate the duration of a cycle of a chest compression phase and a ventilation phase. For example, the processing unit 350 may calculate, for a CPR treatment protocol with 2-rescuers, the time period to complete or perform a CPR cycle of the chest compression phase (e.g., 30 * 0.6 = 18 seconds) and the ventilation phase (e.g., 7 * 0.6 = 4.2 seconds). In other implementations, the ventilation phase may be different (e.g., 8 * 0.6 = 4.8). For the CPR treatment protocol with 1-rescuer, the processing unit 350 may calculate the time period to complete or perform CPR cycle of a chest compression phase (e.g., 30 * 0.6 = 18 seconds) and a ventilation phase (e.g., 14 * 0.6 = 8.4 seconds).

Once the processing unit 350 computes the duration of a cycle of the chest compressions and ventilations, the processing unit 350 may calculate the total time period to perform or complete a CPR treatment based on the number of CPR cycles. In some implementations, when the CPR treatment ends with a ventilation phase, the processing unit 350 may discard the last ventilation phase since ending the CPR treatment with ventilations may be undesirable (e.g., it may effectively decrease the proportion of time with good blood flow, which may have a negative effect on patient outcome). Accordingly, the processing unit 350 may calculate the total time period to perform or complete the CPR treatment based on 5 CPR cycles of the compression phase and 4 CPR cycles of the ventilation phase. For example, the processing unit 350 may compute the total time to perform or complete the CPR treatment for 2-rescuers (e.g., 5 chest compression cycles * 18 seconds + 4 ventilation cycles * 8.4 second = 106.8 seconds) and for 1-rescuser (e.g., 5 chest compression cycles * 18 seconds + 4 ventilation cycles * 4.2 second = 123.6 seconds).

The processing unit 350 may compare the calculated time period to complete the CPR treatment protocol (e.g., 106.8 seconds for 2-rescuers and 123.6 seconds for 1-rescuser) to the received CPR time period (e.g., 120 seconds for the CPR period inputted or selected by the user). When the received CPR time period (e.g., 120 seconds) is less than calculated time period (e.g., 123.6 second) for the CPR treatment protocol (e.g., the CPR treatment protocol for 1 rescuer) resulting in a portion of the chest compressions in the last cycle being discarded or cut-off, the processing unit 350 may adjust the CPR treatment protocol to fit within the received CPR time period (e.g., the CPR time period inputted or selected CRP period). For example, the processing unit 350 may change or decrease the number of chest compressions for one or more of the CPR cycles of the CPR treatment, change or decrease the number of the ventilations for one or more of the CPR cycles of the CPR treatment, adjust or modify (e.g., decrease) a duration or time period of a ventilation of the CPR treatment, adjust or modify (e.g., decrease) the duration or length of the time period between ventilations of the CPR treatment, and/or adjust any other parameter of the CPR protocol to decrease the time period for performing the CPR treatment protocol. The processing unit 350 may be configured to adjust or modify the CPR treatment protocol so that the adjusted or modified CPR treatment protocol remains complaint and consistent with the guidelines of the AHA. In some implementations, the processing unit may modify the CPR treatment protocol by changing a number of chest compressions, a time between chest compressions, or a start time for chest compressions for at least one of the CPR cycles in order to provide an integer number of chest compressions for a CPR time period.

When the received CPR period (120 seconds) is greater than the calculated time period (e.g., 106.8 second) for the CPR treatment protocol (e.g., the CPR treatment protocol for the 2-rescusers), the processing unit 350 may change or increase the number of chest compressions for one or more of the CPR cycles of the CPR treatment, change or increase the number of the ventilations for one or more of the CPR cycles of the CPR treatment, adjust or modify (e.g., increase) a duration or time period of a ventilation of the CPR treatment, adjust or modify (e.g., increase) the duration or length of the time period between ventilations of the CPR treatment, adjust or modify (e.g., decrease) the length or duration of the CPR period inputted or selected by the user, and/or adjust any other parameter of the CPR protocol to increase the time period for the CPR treatment protocol. In some implementations, the processing unit 350 may adjust a ventilation rate (e.g., a 2 second ventilation time period may be adjusted to 2.2 or 2.8 seconds) so that the time period of the CPR treatment protocol approximates the received CPR period. In other implementations, the processing unit 350 may adjust or modify the received CPR period to approximate the calculated time period for the CPR treatment. For example, the processing unit 350 may use the calculated time period for the time period of CPR treatment or make the received CPR period for the CPR treatment equal to the calculated CPR time period. As a result, additional CPR treatments may be applied or delivered immediately after a CPR treatment is delivered to a person. The processing unit 350 may be configured to adjust or modify the CPR treatment protocol so that the adjusted or modified CPR treatment protocol remains complaint and consistent with the guidelines of the AHA. The processing unit 350 may communicate to the user the adjusted or modified CPR treatment for delivering to a person or patient. In some implementations, the processing unit may modify the CPR treatment protocol by changing a number of chest compressions, a time between chest compressions, or a start time for chest compressions for at least one of the CPR cycles in order to provide an integer number of chest compressions for a CPR time period. Thus, the processing unit 350 may override or modify the specific CPR time periods input by a user or rescuer when those inputs are inconsistent with the CPR protocol so that the adjusted or modified CPR treatment protocol provides a best fit for the user inputs (such as an integer number of chest compressions with a CPR time period), satisfies the overall user requirements, and remains complaint and consistent with the guidelines of the AHA.

The processing unit 350 may also receive and evaluate physiological parameters of a person (e.g., electrical activity of the heart) from the sensors 384 and/or the defibrillation electrodes 372 and 374 placed on a person. The processing unit 350 may also determine whether a defibrillation pulse should be delivered to a person based upon the physiological parameters. For example, the processing unit 350 may make a shock/no shock determination based on ECG data and/or other information. In some examples, the processing unit 350 may cause the medical device 302 to automatically deliver defibrillation pulses or may advise the user of these determinations via the user interface 354. When a defibrillation shock has previously been delivered, the processing unit 350 may evaluate the efficacy of the delivered defibrillation pulse by determining if the heart of the person is still fibrillating based on the sensed electrical activity in order to determine whether an additional defibrillation pulse is warranted.

The processing unit 350 may also cause an electrocardiogram (ECG) to be displayed on the user interface 354 that reflects the sensed electrical activity of a heart of a person. Further, the processing unit 350 may determine whether to commence charging of the energy storage module 362. In some embodiments, this processing unit 350 may determine the rate to charge the energy storage module 362. In addition, the processing unit 350 may control delivery of other types of treatment therapy to the person via the defibrillation electrodes 372 and 374, such as cardioversion or pacing therapy.

The processing unit 350 may include one or more general-purpose processors, special purpose processors (e.g., digital signal processors (DSP), application specific integrated circuits (ASIC), graphic processing units, etc.), or any other suitable processing unit or controller. The processing unit 350 can be configured to execute instructions (e.g., computer-readable program instructions) that are stored in memory and may be executable to provide the functionality of the medical device described herein. For example, the processing unit 350 can execute instructions for causing the medical device to display an ECG waveform on the user interface 354 of the medical device 302.

The communication module 356 of the medical device 302 may be in communication with the processing unit 350. The communication module 356 may enable patient data, treatment information, CPR performance, system data, environmental data, etc. to be communicated between the medical device 302 and other devices, such as a remote assistance center and/or any other remote computing device. The communication module 356 may include one or more wireless or wireline interfaces that allow for both short-range communication and long range communication to one or more networks or to one or more remote devices. The wireless interfaces may provide for communication under one or more wireless communication protocols, such as Bluetooth, Wi-Fi (e.g., an institute of electrical and electronic engineers (IEEE) 802.xx protocol), Long-Term Evolution (LTE), cellular communications, near-field communication (NFC), radio-frequency identification (RFID), and/or other wireless communication protocols. Such wireline interfaces may include an Ethernet interface, USB interface, or similar interface to communicate via a wire, a twisted pair of wires, a coaxial cable, an optical link, a fiber-optic link, or other physical connection to a wireline network.

The power source 358 of the medical device 302 may provide power to the components of the medical device 302. The power source 358 may include one or more battery units. The battery units may include lithium batteries, alkaline batteries, nickel cadmium batteries, nickel metal hydride batteries, or any other suitable type of battery or energy device. As shown in Figure 3, the medical device 302 may include a first battery unit 390 and a second battery unit 392. The first battery unit 390 and second battery unit 392 are selectively coupled to the energy storage module 362 by the charging module 360.

The charging module 360 of the medical device 302 may be configured to charge the energy storage module or device 362 before a defibrillation shock is delivered to a person. The charging module 360 may include charging circuitry for charging the energy storage module 362 to a selected voltage level that is determined based on a selected energy level for the defibrillation shock. The energy storage module 362 of the medical device 302 can store electrical energy, in the form of an electrical charge, for delivery of a defibrillation pulse or shock to a person being treated for a medical condition.

The energy storage module 362 can be charged by the charging module 360 to a desired energy level (e.g., between 50 Joules to 360 Joules), as controlled by processing unit 350. In some implementations, the energy storage module 362 may include one or more capacitors 394 that store the energy to be delivered to a person as a defibrillation shock. When the energy of the energy storage module 362 reaches the desired energy level, the defibrillation shock may be delivered manually or automatically.

The discharge circuit 364 of the medical device 302 can enable the energy stored in the energy storage module 362 to be discharged to a person. The electrical energy may be discharged at a selected energy level, via the ports or nodes 376 and 378, to the defibrillation electrodes 372 and 374 to cause a shock to be delivered to the person. The discharge circuit 364 can be controlled by the processing unit 350, or directly by the user via the user interface 354.

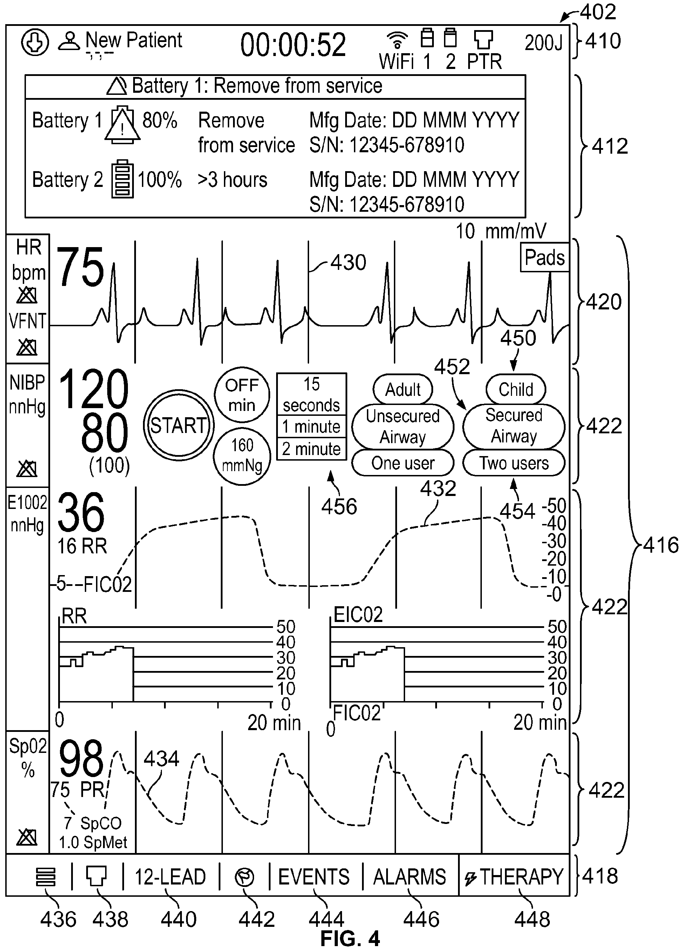

Figure 4 illustrates a GUI 402, in accordance with an example implementation. The GUI 402 can be displayed by a medical device, such as the medical devices of Figures 1-3. The GUI 402 may be configured to allow a user or rescuer to input information (e.g., parameters, variables, etc.) for enabling the medical device to select a cardiac or a resuscitation treatment (e.g., a CPR treatment) for a person experiencing a medical condition. For example, the GUI 402 may allow the user to input or select an age classification of a person 450 (e.g., adult or child/infant), an airway status 452 (e.g., whether the person has his or her airway secured by artificial airway), whether the CPR is being delivered by one or two persons 454, whether the, and/or other variables which may be used by the medical device to determine or select a CPR treatment protocol. The GUI 402 may display user-selectable user-interface items to allow a user to input or select time periods 456 for a CPR treatment

As shown in Figure 4, the GUI 402 includes a status bar 410, a messaging area 412, a content area 416, and a navigation task bar 418. The messaging area 412 of the GUI 402 may display messages and information to the user or rescuer. The content area 416 of the GUI 402 may show patient data including physiologic parameters and waveforms output or displayed by the medical device as well as provided by physiologic sensors. The content area 416 can include multiple channels, including a primary channel 420 for displaying a primary waveform 430 and multiple secondary channels 422 for displaying secondary data, such as a secondary waveform 432..Although multiple secondary channels 422 are shown, in other examples, a GUI may only display a single secondary channel.

The GUI 402 may also be configured to display waveforms next to a side rectangle having a particular color and labelled by a physiologic parameter to which the waveform pertains. For example, the GUI includes the waveform 430 for heart rate (HR), the waveform 432 for End-tidal CO2 (EtCO2), which indicates the partial pressure or maximal concentration of carbon dioxide (CO2) at the end of an exhaled breath, and the waveform 434 for SpO2. The GUI can also display NIBP values for a patient or person being monitored and/or treated. Further, the GUI 402 may display a navigation task bar 418 at the bottom of the GUI 402. The navigation task bar 418 may have various tabs and menu options. As shown, the navigation task bars 418 includes a menu button 436, a print button 438, a 12-Lead button 440, a generic event button 442, an events button 444, an alarms button 446, and therapy button 448.

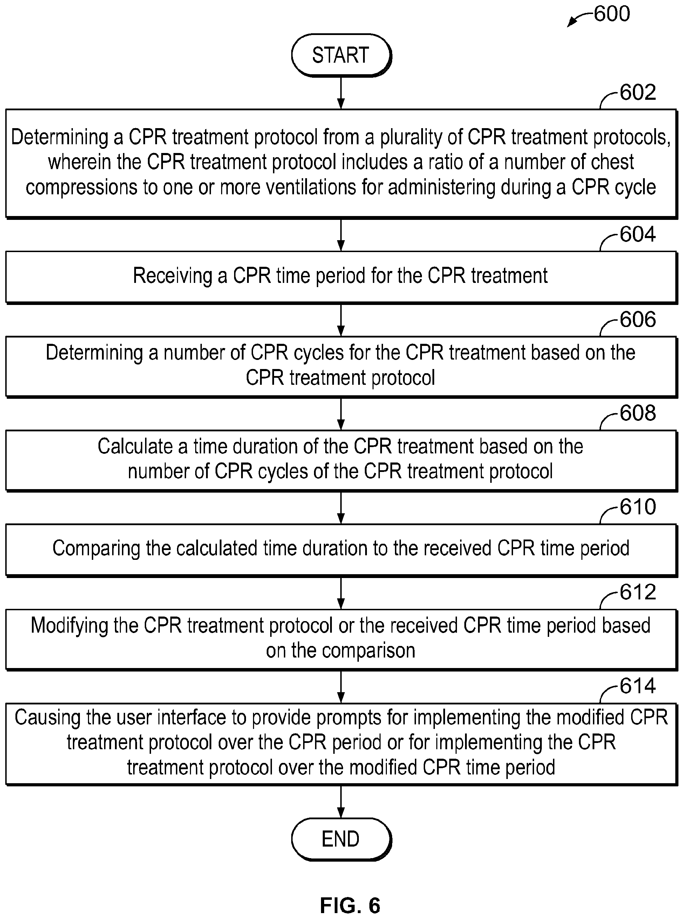

Figure 6 illustrates a flow chart of a method 600 for adjusting a cardiopulmonary resuscitation (CPR) treatment provided by a medical device, according to example implementations. The method 600 represents an example method that may include one or more operations, functions, or actions, as depicted by one or more of blocks 602-614, each of which may be performed by any of the medical devices or systems described herein. For instance, medical devices depicted in Figures 1-3 may enable execution of the method 600.

Those skilled in the art will understand that the flowchart of Figure 6 herein illustrates functionality and operations of certain implementations of the present disclosure. In this regard, each block of the flowchart may represent a module, a segment, or a portion of program code, which includes one or more instructions executable by one or more processors for implementing specific logical functions or steps in the process. The program code may be stored on any type of computer readable medium, for example, such as a storage device including a disk or hard drive.

In addition, each block may represent circuitry that is wired to perform the specific logical functions in the process. Alternative implementations are included within the scope of the example implementations of the present application in which functions may be executed out of order from that shown or discussed, including substantially concurrent or in reverse order, depending on the functionality involved, as would be understood by those reasonably skilled in the art.

At block 602, the method 600 involves determining a CPR treatment protocol from one or more CPR treatment protocols, wherein the CPR treatment protocol includes a ratio of a number of chest compressions to one or more ventilations for administering during a CPR cycle. A medical device may be operated by a user to monitor and provide treatment or care to a person or patient experiencing a medical condition, such as cardiac arrest or any other cardiac rhythm abnormality. The medical device may be the medical device shown in Figures 1-3. The medical device may receive inputs or selections from a user or rescuer relating to an age classification of a person experiencing a medical condition (e.g., adult or child/infant), an airway status (e.g., whether the person has his airway secured by artificial airway), whether the CPR is being delivered by one or two persons, whether the person delivering CPR is a medical professional or a layperson, and/or other variables which may be used by the medical device 102 to determine or select a CPR treatment protocol. The medical device may select a CPR treatment protocol from one or more CPR treatment protocols based on the input received from or selected by the user. The CPR treatment protocol selected by the embodiments may include a pattern or ratio of a number of chest compression to one or more ventilations for a CPR cycle for a CPR treatment (e.g., a number of chest compressions followed by one or more ventilations during a CPR cycle).

At block 604, the method 600 involves receiving a CPR time period for the CPR treatment. The medical device may receive information relating to a medical treatment, such as a CPR time period for performing a CPR treatment (e.g., 15 seconds, 1 minute or 60 seconds, 2 minutes or 120 seconds, 3 minutes or 180 seconds, etc.). For example, the medical device may receive a 120-second CPR time period inputted or selected by a user of the medical device.

At block 606, the method 600 involves determining a number of CPR cycles for the CPR treatment based on the CPR treatment protocol. The medical device may determine the number of cycles of chest compressions and ventilations to be performed. For example, the medical device may be configured to select an integer number of CPR cycles of chest compressions and ventilations to be performed during a CPR time period inputted or selected by the user for the CPR treatment.

At block 608, the method 600 involves calculating a time duration of the CPR treatment protocol based on the number of CPR cycles of the CPR treatment protocol. Once the medical device computes the duration of a cycle of the chest compressions and ventilations, the medical device may calculate the total time period to perform or complete a CPR treatment based on the number of CPR cycles. For example, the medical device may calculate the total time period to perform or complete the CPR treatment based on the number of cycles of the compression phase and the number of cycles of the ventilation phase.

At block 610, the method involves comparing the calculated time duration to the received CPR time period. The medical device may compare a calculated time period to perform or complete the CPR treatment protocol to the received CPR period.

At block 612, the method involves modifying the CPR treatment or the received CPR time period based on the comparison. The medical device may modify or adjust the CPR treatment protocol based on the received CPR period (e.g., user inputted or selected CRP time period). For example, the medical may change the number of chest compressions for one or more of the CPR cycles of the CPR treatment, change the number of the ventilations for one or more of the CPR cycles of the CPR treatment, adjust or modify a duration or time period of a ventilation of the CPR treatment, and/or adjust or modify the duration or length of the time period between ventilations of the CPR treatment. In some implementations, the medical device may adjust or modify the received CPR period to approximately the calculated time period for the CPR treatment.

At block 614, the method involves causing the user interface to provide prompts for implementing the modified CPR treatment protocol over the CPR period or for implementing the CPR treatment protocol over the modified CPR time period. The medical device may communication the CPR treatment and/or the modified CPR treatment to the user of the medical device. The communicated information may include at least chest compression instructions. The CPR treatment may also be displayed by the medical device.
Clause 1. A portable medical device for adjusting a cardiopulmonary resuscitation (CPR) treatment, the portable medical device comprising:
   a user interface configured to provide prompts to a user;
   a memory device configured to store one or more CPR treatment protocols; and
   a processor configured to:
      determine a particular CPR treatment protocol from the one or more CPR treatment protocols, wherein the particular CPR treatment protocol indicates a ratio of chest compressions to ventilations for administering during a CPR cycle;
      receive an indication of a CPR time period for the CPR treatment;
      determine a number of CPR cycles for the CPR treatment based on the particular CPR treatment protocol;
      calculate a time duration of the CPR treatment based on the number of CPR cycles of the particular CPR treatment protocol;
      compare the time duration to the CPR time period;
      modify the particular CPR treatment protocol or the CPR time period based on a comparison between the time duration and the CPR time period; and
      cause the user interface to provide prompts for:
         implementing a modified version of the particular CPR treatment protocol over the CPR time period; or
         implementing the particular CPR treatment protocol over a modified CPR time period.
Clause 2. The portable medical device of clause 1, wherein the CPR time period is input by the user or selected by the user.
Clause 3. The portable medical device of clause 1 or 2, wherein the CPR time period is modified when the CPR time period is greater than the time duration of the CPR treatment.
Clause 4. The portable medical device of clause 1, 2 or 3, wherein a duration of the CPR time period is modified to be equal to the time duration of the CPR treatment.
Clause 5. The portable medical device of any of clauses 1-4, wherein the particular CPR treatment protocol is modified when the CPR time period is less than the time duration of the CPR treatment.
Clause 6. The portable medical device of any of clauses 1-5, wherein modifying the particular CPR treatment protocol includes changing a number of chest compressions, changing a time between chest compressions, or changing a start time for chest compressions for at least one CPR cycle in order to provide an integer number of chest compressions within the CPR time period.
Clause 7. The portable medical device of any of clauses 1-6, wherein modifying the particular CPR treatment protocol includes changing a number of the ventilations, changing a duration or time period of at least ventilation, changing a time between ventilations, changing a start time for ventilations, or changing a length or duration of a time period between ventilations for at least one CPR cycle.
Clause 8. The portable medical device of any of clauses 1-7, wherein the particular CPR treatment protocol is determined based on an age classification of a patient, a patient airway status, a number of persons delivering CPR to the patient, whether a medical professional or a layperson is delivering CPR, or a combination thereof.
Clause 9. The portable medical device of any of clauses 1-8, wherein the processor is further configured to cause the user interface to provide, based on the particular CPR treatment protocol, prompts at a first rate of chest compressions or ventilations during a modified version of the CPR time period.
Clause 10. The portable medical device of any of clauses 1-9, wherein the prompts include a voice prompt, an audio prompt, or a combination thereof.
Clause 11. The portable medical device of any of clauses 1-10, wherein the processor is further configured to cause the user interface to provide, based on the modified CPR treatment protocol, prompts at a first rate of chest compressions or ventilations during the CPR time period.
Clause 12. The portable medical device of any of clauses 1-12, further comprising a connection port configured to provide a high energy output to one or more defibrillator electrodes.
Clause 13. The portable medical device of any of clauses 1-12, further comprising a defibrillator assembly or a patient monitoring device.
Clause 14. A method for adjusting a cardiopulmonary resuscitation (CPR) treatment provided by a defibrillator, the method comprising:
   determining, by one or more processors of the defibrillator, a particular CPR treatment protocol from one or more CPR treatment protocols, wherein the particular CPR treatment protocol indicates a ratio of chest compressions to ventilations for administering during a CPR cycle;
   receiving, by the one or more processors, an indication of a CPR time period for the CPR treatment;
   determining, by the one or more processors, a number of CPR cycles for the CPR treatment based on the particular CPR treatment protocol;
   calculating, by the one or more processors, a time duration of the CPR treatment based on the number of CPR cycles of the particular CPR treatment protocol;
   comparing, by the one or more processors, the time duration to the CPR time period;
   modifying the particular CPR treatment protocol or the CPR time period based on a comparison between the time duration and the CPR time period; and
   causing a user interface to provide prompts for:
      implementing a modified version of the particular CPR treatment protocol over the CPR time period; or
      implementing the particular CPR treatment protocol over a modified CPR time period.
Clause 15. The method of clause 14, wherein the CPR time period is input by the user or selected by the user.
Clause 16. The method of clause 14 or 15, wherein the CPR time period is modified when the CPR time period is greater than the time duration of the CPR treatment.
Clause 17. The method of clause 14, 15 or 16, wherein a duration of the CPR time period is modified to be equal to the time duration of the CPR treatment.
Clause 18. The method of any of clauses 14-17, wherein the particular CPR treatment protocol is modified when the CPR time period is less than the time duration of the CPR treatment.
Clause 19. A non-transitory computer-readable medium comprising instructions that, when executed by a processor of defibrillator, cause the processor to perform operations for adjusting a cardiopulmonary resuscitation (CPR) treatment, the operations comprising:
   determining a particular CPR treatment protocol from one or more CPR treatment protocols, wherein the particular CPR treatment protocol indicates a ratio of chest compressions to ventilations for administering during a CPR cycle;
   receiving an indication of a CPR time period for the CPR treatment;
   determining a number of CPR cycles for the CPR treatment based on the particular CPR treatment protocol;
   calculating a time duration of the CPR treatment based on the number of CPR cycles of the particular CPR treatment protocol;
   comparing the time duration to the CPR time period;
   modifying the particular CPR treatment protocol or the CPR time period based on a comparison between the time duration and the CPR time period; and
   causing a user interface to provide prompts for:
      implementing a modified version of the particular CPR treatment protocol over the CPR time period; or
      implementing the particular CPR treatment protocol over a modified CPR time period.
Clause 20. The non-transitory computer-readable medium of clause 19, wherein the prompts include a voice prompt, an audio prompt, or a combination thereof.

The embodiments described herein can be realized in hardware, software, or a combination of hardware and software. For example, the embodiments can be realized in a centralized fashion in at least one computer system or in a distributed fashion where different elements are spread across interconnected computer systems. Any kind of computer system or other apparatus adapted for carrying out the methods described herein can be employed. Further, the embodiments described herein can be embedded in a computer program product, which includes all the features enabling the implementation of the operations described herein and which, when loaded in a computer system, can carry out these operations.

The flowcharts and block diagrams described herein illustrate the architecture, functionality, and operation of possible implementations of systems, methods, and apparatus according to various illustrative embodiments. In this regard, each block in the flowcharts or block diagrams may represent a module, segment, or portion of code, which comprises one or more executable instructions for implementing the specified logical function or functions. It should also be noted that, in some alternative implementations, the functions noted in a block may occur out of the order noted in the drawings. For example, the functions of two blocks shown in succession may be executed substantially concurrently, or the functions of the blocks may sometimes be executed in the reverse order, depending upon the functionality involved.

Additionally, instances in this specification where one element is "coupled" to another element can include direct and indirect coupling. Direct coupling can be defined as one element coupled to and in some contact with another element. Indirect coupling can be defined as coupling between two elements not in direct contact with each other, but having one or more additional elements between the coupled elements. Further, as used herein, securing one element to another element can include direct securing and indirect securing. Additionally, as used herein, "adjacent" does not necessarily denote contact. For example, one element can be adjacent another element without being in contact with that element.

As used herein, a system, apparatus, structure, article, element, component, or hardware "configured to" perform a specified function is indeed capable of performing the specified function without any alteration, rather than merely having potential to perform the specified function after further modification. In other words, the system, apparatus, structure, article, element, component, or hardware "configured to" perform a specified function is specifically selected, created, implemented, utilized, programmed, and/or designed for the purpose of performing the specified function. As used herein, "configured to" denotes existing characteristics of a system, apparatus, structure, article, element, component, or hardware which enable the system, apparatus, structure, article, element, component, or hardware to perform the specified function without further modification. For purposes of this disclosure, a system, apparatus, structure, article, element, component, or hardware described as being "configured to" perform a particular function may additionally or alternatively be described as being "adapted to" and/or as being "operative to" perform that function.

By the term "substantially" and "about" used herein, it is meant that the recited characteristic, parameter, or value need not be achieved exactly, but that deviations or variations, including for example, tolerances, measurement error, measurement accuracy limitations and other factors known to skill in the art, may occur in amounts that do not preclude the effect the characteristic was intended to provide.

Unless otherwise indicated, the terms "first," "second," etc. are used herein merely as labels, and are not intended to impose ordinal, positional, or hierarchical requirements on the items to which these terms refer. Moreover, reference to, e.g., a "second" item does not require or preclude the existence of, e.g., a "first" or lower-numbered item, and/or, e.g., a "third" or higher-numbered item.

While apparatus has been described with reference to certain examples, it will be understood by those skilled in the art that various changes can be made and equivalents can be substituted without departing from the scope of the claims. Therefore, it is intended that the present apparatus not be limited to the particular examples disclosed, but that the disclosed apparatus include all embodiments falling within the scope of the appended claims.

## Claims

1. A portable medical device for adjusting a cardiopulmonary resuscitation (CPR) treatment, the portable medical device comprising:
a user interface configured to provide prompts to a user;
a memory device configured to store one or more CPR treatment protocols; and
a processor configured to:
determine a particular CPR treatment protocol from the one or more CPR treatment protocols, wherein the particular CPR treatment protocol indicates a ratio of chest compressions to ventilations for administering during a CPR cycle;
receive an indication of a CPR time period for the CPR treatment;
determine a number of CPR cycles for the CPR treatment based on the particular CPR treatment protocol;
calculate a time duration of the CPR treatment based on the number of CPR cycles of the particular CPR treatment protocol;
compare the time duration to the CPR time period;
modify the particular CPR treatment protocol or the CPR time period based on a comparison between the time duration and the CPR time period; and
cause the user interface to provide prompts for:
implementing a modified version of the particular CPR treatment protocol over the CPR time period; or
implementing the particular CPR treatment protocol over a modified CPR time period.

2. The portable medical device of claim 1, wherein the CPR time period is input by the user or selected by the user.

3. The portable medical device of claim 1 or 2, wherein a duration of the CPR time period is modified to be equal to the time duration of the CPR treatment.

4. The portable medical device of claim 1, 2 or 3, wherein modifying the particular CPR treatment protocol includes changing a number of chest compressions, changing a time between chest compressions, or changing a start time for chest compressions for at least one CPR cycle in order to provide an integer number of chest compressions within the CPR time period.

5. The portable medical device of any of claims 1-4, wherein modifying the particular CPR treatment protocol includes changing a number of the ventilations, changing a duration or time period of at least ventilation, changing a time between ventilations, changing a start time for ventilations, or changing a length or duration of a time period between ventilations for at least one CPR cycle.

6. The portable medical device of any of claims 1-5, wherein the particular CPR treatment protocol is determined based on an age classification of a patient, a patient airway status, a number of persons delivering CPR to the patient, whether a medical professional or a layperson is delivering CPR, or a combination thereof.

7. The portable medical device of any of claims 1-6, wherein the processor is further configured to cause the user interface to provide, based on the particular CPR treatment protocol, prompts at a first rate of chest compressions or ventilations during a modified version of the CPR time period.

8. The portable medical device of any of claims 1-7, wherein the prompts include a voice prompt, an audio prompt, or a combination thereof.

9. The portable medical device of any of claims 1-8, wherein the processor is further configured to cause the user interface to provide, based on the modified CPR treatment protocol, prompts at a first rate of chest compressions or ventilations during the CPR time period.

10. The portable medical device of any of claims 1-9, further comprising a connection port configured to provide a high energy output to one or more defibrillator electrodes.

11. The portable medical device of any of claims 1-10, further comprising a defibrillator assembly or a patient monitoring device.

12. A method for adjusting a cardiopulmonary resuscitation (CPR) treatment provided by a defibrillator, the method comprising:
determining, by one or more processors of the defibrillator, a particular CPR treatment protocol from one or more CPR treatment protocols, wherein the particular CPR treatment protocol indicates a ratio of chest compressions to ventilations for administering during a CPR cycle;
receiving, by the one or more processors, an indication of a CPR time period for the CPR treatment;
determining, by the one or more processors, a number of CPR cycles for the CPR treatment based on the particular CPR treatment protocol;
calculating, by the one or more processors, a time duration of the CPR treatment based on the number of CPR cycles of the particular CPR treatment protocol;
comparing, by the one or more processors, the time duration to the CPR time period;
modifying the particular CPR treatment protocol or the CPR time period based on a comparison between the time duration and the CPR time period; and
causing a user interface to provide prompts for:
implementing a modified version of the particular CPR treatment protocol over the CPR time period; or
implementing the particular CPR treatment protocol over a modified CPR time period.

13. The method of claim 12, wherein the CPR time period is modified when the CPR time period is greater than, or less than, the time duration of the CPR treatment.

14. The method of claim 12 or 13, wherein a duration of the CPR time period is modified to be equal to the time duration of the CPR treatment.

15. A non-transitory computer-readable medium comprising instructions that, when executed by a processor of defibrillator, cause the processor to perform operations for adjusting a cardiopulmonary resuscitation (CPR) treatment, the operations comprising:
determining a particular CPR treatment protocol from one or more CPR treatment protocols, wherein the particular CPR treatment protocol indicates a ratio of chest compressions to ventilations for administering during a CPR cycle;
receiving an indication of a CPR time period for the CPR treatment;
determining a number of CPR cycles for the CPR treatment based on the particular CPR treatment protocol;
calculating a time duration of the CPR treatment based on the number of CPR cycles of the particular CPR treatment protocol;
comparing the time duration to the CPR time period;
modifying the particular CPR treatment protocol or the CPR time period based on a comparison between the time duration and the CPR time period; and
causing a user interface to provide prompts for:
implementing a modified version of the particular CPR treatment protocol over the CPR time period; or
implementing the particular CPR treatment protocol over a modified CPR time period.
